(19) Europäisches Patentamt
European Patent Office
Office européen des brevets

(11) **EP 4 212 037 A1**

(12) **EUROPEAN PATENT APPLICATION**
published in accordance with Art. 153(4) EPC

(43) Date of publication:
**19.07.2023   Bulletin 2023/29**

(21) Application number: **20965879.8**

(22) Date of filing: **15.12.2020**

(51) International Patent Classification (IPC):
***A24F 40/50*** *(2020.01)*

(52) Cooperative Patent Classification (CPC):
**A24F 40/50**

(86) International application number:
**PCT/JP2020/046702**

(87) International publication number:
**WO 2022/130491 (23.06.2022 Gazette 2022/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Japan Tobacco Inc.
Tokyo 105-6927 (JP)**

(72) Inventors:
• **KAWANAGO, Hiroshi
Tokyo 130-8603 (JP)**

• **FUJIKI, Takashi
Tokyo 130-8603 (JP)**
• **AOYAMA, Tatsunari
Tokyo 130-8603 (JP)**
• **YOSHIDA, Ryo
Tokyo 130-8603 (JP)**

(74) Representative: **Hoffmann Eitle
Patent- und Rechtsanwälte PartmbB
Arabellastraße 30
81925 München (DE)**

(54) **INHALATION DEVICE AND CONTROL METHOD**

(57)     [Problem] To provide a mechanism by which it is possible to improve operational safety regarding a power source of an inhalation device.

[Solution] This inhalation device generates an aerosol, and comprises: a first resistor having a prescribed resistance; a measurement subject with variable resistance that is connected in series to the first resistor; an A/D converter that outputs an A/D conversion value of an applied voltage; and a measurement unit that measures the resistance of the measurement subject on the basis of the A/D conversion value outputted from the A/D converter. The A/D converter has applied thereto a reference voltage from a first power source, the reference voltage being a voltage that determines a full scale of the A/D conversion value, outputs an A/D conversion value of a first voltage applied from a second power source differing from the first power source, and outputs an A/D conversion value of a second voltage applied to the measurement subject from the second power source via the first resistor. The measurement unit calculates the resistance of the measurement subject on the basis of the ratio of the A/D conversion value of the first voltage and the A/D conversion value of the second voltage, and of the resistance of the first resistor

FIG. 3

**Description**

Technical Field

The present invention relates to an inhaler device and a control method.

Background Art

Inhaler devices, such as e-cigarettes and nebulizers, that generate material to be inhaled by a user are widespread. For example, an inhaler device generates an aerosol having a flavor component imparted thereto, by using a substrate including an aerosol source for generating the aerosol, a flavor source for imparting the flavor component to the generated aerosol, and the like. The user is able to enjoy the flavor by inhaling the aerosol having the flavor component imparted thereto, which is generated by the inhaler device.

In recent years, it has been studied to mount various types of power supplies in inhaler devices. For example, in Patent Literature 1 below, it is studied to mount a lithium ion battery, a solid-state battery, or the like in an inhaler device.

Citation List

Patent Literature

Patent Literature 1: JP 2020-504599 A

Summary of Invention

Technical Problem

However, the technique disclosed in Patent Literature 1 has only a short history since its development, and has room for improvement from various viewpoints.

The present invention has been made in view of the above problem, and an object of the present invention is to provide a mechanism capable of improving the stability of operation regarding a power supply in an inhaler device.

Solution to Problem

To solve the above problem, according to an aspect to the present invention, there is provided an inhaler device configured to generate an aerosol. The inhaler device includes a first resistor having a predetermined resistance, a measurement target connected in series to the first resistor and having a resistance that is variable, an analog-to-digital (A/D) converter configured to output an A/D conversion value of a voltage applied thereto, and a measurer configured to measure the resistance of the measurement target, based on the A/D conversion value output from the A/D converter. The A/D converter is configured to receive a reference voltage from a first power supply, the reference voltage being a voltage determining a full scale of an A/D conversion value; output an A/D conversion value of a first voltage applied from a second power supply different from the first power supply; and output an A/D conversion value of a second voltage applied to the measurement target from the second power supply via the first resistor. The measurer is configured to calculate the resistance of the measurement target, based on a ratio between the A/D conversion value of the first voltage and the A/D conversion value of the second voltage, and on the resistance of the first resistor.

The measurer may be configured to calculate the resistance of the measurement target by using an equation below:

[Math. 1]

$$Rdut = \frac{R \times \dfrac{\text{A/D conversion value } (Vad)}{\text{A/D conversion value } (Vdut)}}{1 - \dfrac{\text{A/D conversion value } (Vad)}{\text{A/D conversion value } (Vdut)}} \quad \cdots (1)$$

,

where A/D conversion value (Vdut) is the A/D conversion value of the first voltage, A/D conversion value (Vad) is the A/D conversion value of the second voltage, R is the resistance of the first resistor, and Rdut is the resistance of the measurement target.

The first voltage may have an absolute value that is equal to or smaller than an absolute value of the reference voltage.

The inhaler device may further include a corrector configured to perform correction such that the absolute value of the first voltage is equal to or smaller than the absolute value of the reference voltage.

The corrector may be a second resistor having a predetermined resistance.

The corrector may be an amplifier configured to attenuate a voltage input thereto and output the attenuated voltage.

The A/D converter may be configured to output the A/D conversion value of the first voltage applied to the first resistor from the second power supply.

The measurer may be configured to measure a temperature of the measurement target, based on the resistance of the measurement target.

The inhaler device may further include a heater configured to heat an aerosol source to generate the aerosol. The measurement target may be the heater.

The inhaler device may further include a heater configured to heat an aerosol source to generate the aerosol, and a thermistor having a temperature that changes in accordance with a temperature change of the heater. The measurement target may be the thermistor.

Supply of electric power to the measurement target, for measuring the resistance of the measurement target, may be periodically executed at a first timing. Supply of electric power to the heater, for heating the aerosol source by the heater, may be periodically executed at a second timing different from the first timing.

To solve the above problem, according to another aspect to the present invention, there is provided a control method for controlling an inhaler device configured to generate an aerosol. The inhaler device includes a first resistor having a predetermined resistance, a measurement target connected in series to the first resistor and having a resistance that is variable, and an analog-to-digital (A/D) converter configured to output an A/D conversion value of a voltage applied thereto. The control method includes applying a reference voltage to the A/D converter from a first power supply, the reference voltage being a voltage determining a full scale of an A/D conversion value; applying a first voltage to the A/D converter from a second power supply different from the first power supply and causing the A/D converter to output an A/D conversion value of the first voltage; applying a second voltage to the measurement target from the second power supply via the first resistor and causing the A/D converter to output an A/D conversion value of the second voltage; and calculating the resistance of the measurement target, based on a ratio between the A/D conversion value of the first voltage and the A/D conversion value of the second voltage, and on the resistance of the first resistor.

Advantageous Effects of Invention

As described above, according to the present invention, a mechanism capable of improving the stability of operation regarding a power supply in an inhaler device is provided.

Brief Description of Drawings

[Fig. 1] Fig. 1 is a schematic diagram of an inhaler device according to a first configuration example.

[Fig. 2] Fig. 2 is a schematic diagram of an inhaler device according to a second configuration example.

[Fig. 3] Fig. 3 is a diagram for describing a configuration related to A/D conversion of an inhaler device according to the present embodiment.

[Fig. 4] Fig. 4 is a diagram for describing an example of a timing to supply electric power to a heater in the inhaler device according to the present embodiment.

[Fig. 5] Fig. 5 is a flowchart illustrating an example of a flow of a process executed by the inhaler device according to the present embodiment.

[Fig. 6] Fig. 6 is a diagram for describing a configuration related to A/D conversion of an inhaler device according to a comparative example.

Description of Embodiments

Hereinafter, a preferred embodiment of the present invention will be described in detail with reference to the accompanying drawings. In the specification and the drawings, structural elements having substantially the same functional configuration are denoted by the same reference numerals, and a duplicate description will be omitted.

In the specification and the drawings, elements having substantially the same functional configuration may be distinguished from each other by attaching different alphabetic characters after the same reference numerals. For example, a plurality of elements having substantially the same functional configuration are distinguished as inhaler devices 100A and 100B as necessary. However, when it is not necessary to particularly distinguish each of a plurality of elements having substantially the same functional configuration, only the same reference numeral is given. For example, inhaler devices 100A and 100B are simply referred to as inhaler devices 100 when there is no particular needs to distinguish between them.

«1. Configuration example of inhaler device»

An inhaler device generates material to be inhaled by a user. In the example described below, the material generated by the inhaler device is an aerosol. Alternatively, the material generated by the inhaler device may be gas.

(1) First configuration example

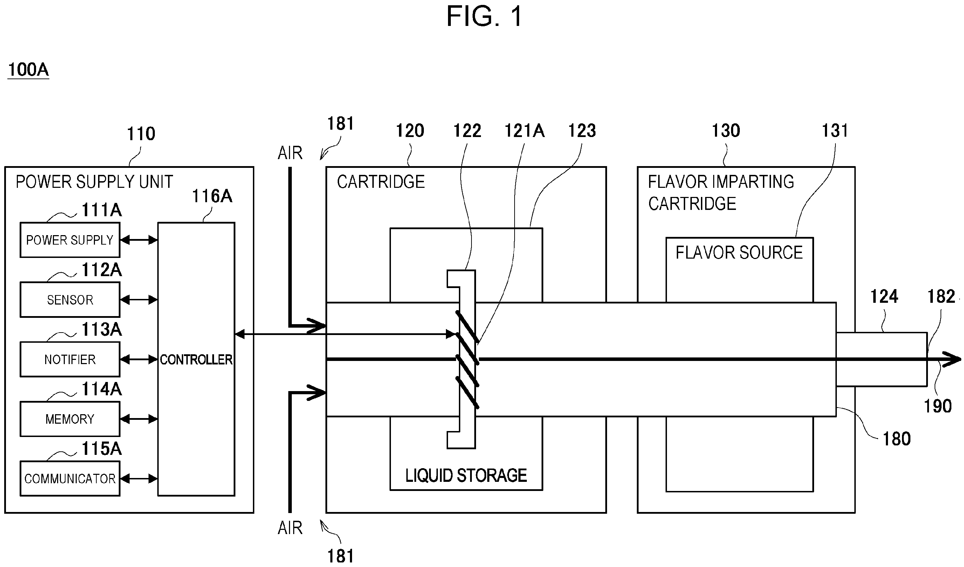

Fig. 1 is a schematic diagram of the inhaler device according to the first configuration example. As illustrated in Fig. 1, an inhaler device 100A according to the present configuration example includes a power supply unit 110, a cartridge 120, and a flavor imparting cartridge 130. The power supply unit 110 includes a power supply 111A, a sensor 112A, a notifier 113A, a memory 114A, a communicator 115A, and a controller 116A. The cartridge 120 includes a heater 121A, a liquid guide 122, and a liquid storage 123. The flavor imparting cartridge 130 includes a flavor source 131 and a mouthpiece 124. In the cartridge 120 and the flavor imparting cartridge 130, an airflow path 180 is defined.

The power supply 111A stores electric power. The power supply 111A supplies electric power to the structural elements of the inhaler device 100A under the control of the controller 116A. The power supply 111A may be a rechargeable battery such as a lithium ion secondary battery.

The sensor 112A acquires various items of information regarding the inhaler device 100A. In an example, the sensor 112A may be a pressure sensor such as a condenser microphone, a flow sensor, or a temperature sensor, and acquire a value generated in accordance with the user's inhalation. In another example, the sensor 112A may be an input device that receives information input by the user, such as a button or a switch.

The notifier 113A provides information to the user. The notifier 113A may be a light-emitting device that emits light, a display device that displays an image, a sound output device that outputs sound, or a vibration device that vibrates.

The memory 114A stores various items of information for operation of the inhaler device 100A. The memory 114A may be a non-volatile storage medium such as flash memory.

The communicator 115A is a communication interface capable of communication in conformity with any wired or wireless communication standard. Such a communication standard may be, for example, Wi-Fi (registered trademark) or Bluetooth (registered trademark).

The controller 116A functions as an arithmetic processing unit and a control circuit, and controls the overall operations of the inhaler device 100A in accordance with various programs. The controller 116A includes an electronic circuit such as a central processing unit (CPU) or a microprocessor, for example.

The liquid storage 123 stores an aerosol source. The aerosol source is atomized to generate an aerosol. The aerosol source is a liquid such as polyhydric alcohol or water. Examples of the polyhydric alcohol include glycerine and propylene glycol. The aerosol source may include a flavor component that is either derived from tobacco or not derived from tobacco. For the inhaler device 100A that is a medical inhaler such as a nebulizer, the aerosol source may include a medicine.

The liquid guide 122 guides, from the liquid storage 123, the aerosol source that is the liquid stored in the liquid storage 123, and holds the aerosol source. The liquid guide 122 is, for example, a wick formed by twining fiber material such as glass fiber or porous material such as porous ceramic. In this case, the capillary action of the wick guides the aerosol source stored in the liquid storage 123.

The heater 121A heats the aerosol source to atomize the aerosol source and generate the aerosol. In the example illustrated in Fig. 1, the heater 121A includes a coil wound around the liquid guide 122. When the heater 121A produces heat, the aerosol source held by the liquid guide 122 is heated and atomized to generate the aerosol. The heater 121A produces heat when receiving electric power from the power supply 111A. In an example, the electric power may be supplied in response to the sensor 112A detecting a start of the user's inhalation and/or an input of predetermined information. Subsequently, the supply of the electric power may be stopped in response to the sensor 112A detecting an end of the user's inhalation and/or an input of predetermined information.

The flavor source 131 is a structural element for imparting a flavor component to the aerosol. The flavor source 131 may include a flavor component that is either derived from tobacco or not derived from tobacco.

The airflow path 180 is a flow path of air to be inhaled by the user. The airflow path 180 has a tubular structure having an air inlet hole 181 and an air outlet hole 182 at both ends. The air inlet hole 181 is an inlet of air into the airflow path 180, and the air outlet hole 182 is an outlet of the air from the airflow path 180. The liquid guide 122 is on the airflow path 180 at an upstream position (closer to the air inlet hole 181), and the flavor source 131 is on the airflow path 180 at a downstream position (closer to the air outlet hole 182). Air flowing in through the air inlet hole 181 when the user inhales mixes with the aerosol generated by the heater 121A. Subsequently, as indicated by an arrow 190, the mixture fluid of the aerosol and the air passes through the flavor source 131 and is conveyed to the air outlet hole 182. When the mixture fluid of the aerosol and the air passes through the flavor source 131, the flavor component included in the flavor source 131 is imparted to the aerosol.

The mouthpiece 124 is to be held in a mouth of the user during inhalation. The mouthpiece 124 has the air outlet hole 182. When the user inhales with the mouthpiece 124 in his/her mouth, the mixture fluid of the aerosol and the air enters the oral cavity of the user.

The configuration example of the inhaler device 100A has been described above. The inhaler device 100A is not limited to the above configuration, and may be configured in various ways as exemplified below.

In an example, the inhaler device 100A does not have to include the flavor imparting cartridge 130. In this case, the cartridge 120 includes the mouthpiece 124.

In another example, the inhaler device 100A may include various types of aerosol sources. Still another type of aerosol may be generated by mixing a plurality of types of aerosols generated from the plurality of types of aerosol sources in the airflow path 180 and causing a chemical reaction.

In addition, means for atomizing the aerosol source is not limited to heating by the heater 121A. For example, the means for atomizing the aerosol source may be vibration atomization or induction heating.

(2) Second configuration example

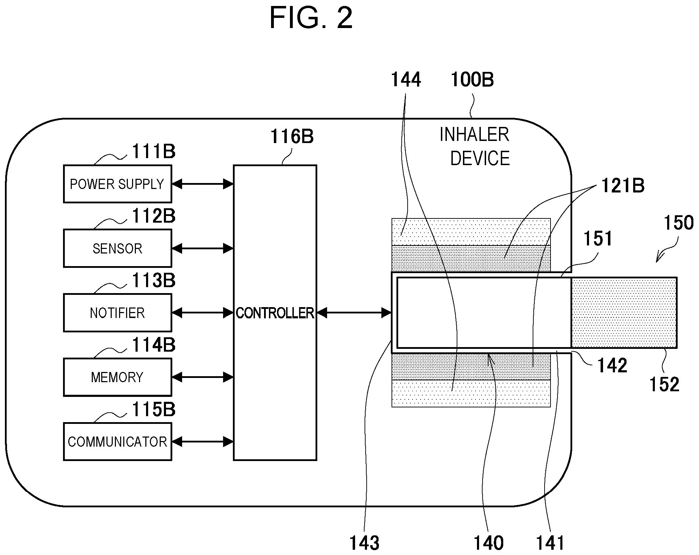

Fig. 2 is a schematic diagram of the inhaler device according to the second configuration example. As illustrated in Fig. 2, an inhaler device 100B according to the present configuration example includes a power supply 111B, a sensor 112B, a notifier 113B, a memory 114B, a communicator 115B, a controller 116B, a heater 121B, a holder 140, and a heat insulator 144.

The power supply 111B, the sensor 112B, the notifier 113B, the memory 114B, the communicator 115B, and the controller 116B are substantially the same as the respective corresponding structural elements included in the inhaler device 100A according to the first configuration example.

The holder 140 has an internal space 141, and holds a stick substrate 150 in a manner partially accommodated in the internal space 141. The holder 140 has an opening 142 that allows the internal space 141 to communicate with outside. The holder 140 holds the stick substrate 150 that is inserted into the internal space 141 through the opening 142. For example, the holder 140 may be a tubular body having the opening 142 and a bottom 143 on its ends, and may define the pillar-shaped internal space 141. The holder 140 also has a function of defining a flow path of air supplied to the stick substrate 150. An air inlet hole, which is an inlet of air to the flow path, is disposed in the bottom 143, for example. On the other hand, an air outlet hole, which is an outlet of air from the flow path, is the opening 142.

The stick substrate 150 includes a substrate 151 and an inhalation port 152. The substrate 151 includes an aerosol source. In the present configuration example, the aerosol source is not limited to a liquid, and may be a solid. The stick substrate 150 held by the holder 140 includes the substrate 151 at least partially accommodated in the internal space 141 and the inhalation port 152 at least partially protruding from the opening 142. When the user inhales with the inhalation port 152 protruding from the opening 142 in his/her mouth, air flows into the internal space 141 through the air inlet hole (not illustrated), and the air and an aerosol generated from the substrate 151 reach inside the mouth of the user.

The heater 121B has a configuration similar to that of the heater 121A according to the first configuration example. However, in the example illustrated in Fig. 2, the heater 121B has a film-like shape and surrounds the outer circumference of the holder 140. Subsequently, heat produced from the heater 121B heats the substrate 151 of the stick substrate 150 from the outer circumference, generating the aerosol.

The heat insulator 144 prevents heat from transferring from the heater 121B to the other structural elements. For example, the heat insulator 144 may be a vacuum heat insulator or an aerogel heat insulator.

The configuration example of the inhaler device 100B has been described above. The inhaler device 100B is not limited to the above configuration, and may be configured in various ways as exemplified below.

In an example, the heater 121B may have a blade-like shape, and may be disposed so that the heater 121B protrudes from the bottom 143 of the holder 140 toward the internal space 141. In this case, the heater 121B having the blade-like shape is inserted into the substrate 151 of the stick substrate 150 and heats the substrate 151 of the stick substrate 150 from its inside. In another example, the heater 121B may be disposed so that the heater 121B covers the bottom 143 of the holder 140. In still another example, the heater 121B may be implemented as a combination of two or more selected from a first heater that covers the outer circumference of the holder 140, a second heater having the blade-like shape, and a third heater that covers the bottom 143 of the holder 140.

In another example, the holder 140 may include an opening/closing mechanism that at least partially opens and closes an outer shell defining the internal space 141. Examples of the opening/closing mechanism include a hinge. In addition, the holder 140 may sandwich the stick substrate 150 inserted into the internal space 141 by opening and closing the outer shell. In this case, the heater 121B may be at the sandwiching position of the holder 140 and may produce heat while pressing the stick substrate 150.

In addition, means for atomizing the aerosol source is not limited to heating by the heater 121B. For example, the means for atomizing the aerosol source may be induction heating.

In addition, the inhaler device 100B may also include the heater 121A, the liquid guide 122, the liquid storage 123, and the airflow path 180 according to the first configuration example. The air outlet hole 182 of the airflow path 180 may also serve as an air inlet hole to the internal space 141. In this case, a mixture fluid of the air and an aerosol generated by the heater 121A flows into the internal space 141, mixes further with an aerosol generated by the heater 121B, and then reaches the oral cavity of the user.

<<2. Technical features>>

(1) Technical issues

Analog-to-digital (A/D) conversion is performed in the inhaler device 100. In a typical power supply configuration related to A/D conversion, a reference voltage VREF for determining the full scale of an A/D conversion value and a voltage VDUT applied to a circuit to be measured are supplied from the same power supply. This is because, when a voltage supplied from the power supply varies, the voltage VDUT varies in accordance with a variation in the reference voltage VREF, so that a tracking operation works and a full-scale variation in the A/D conversion value is cancelled out.

On the other hand, when the reference voltage VREF and the voltage VDUT are supplied from different power supplies, the voltage value of the voltage VDUT can be determined independently of the reference voltage VREF, and thus highly accurate A/D conversion can be performed. In this case, however, the above-described tracking operation does not work, and thus influences of variations in the reference voltage VREF and the voltage VDUT appear in the A/D conversion value.

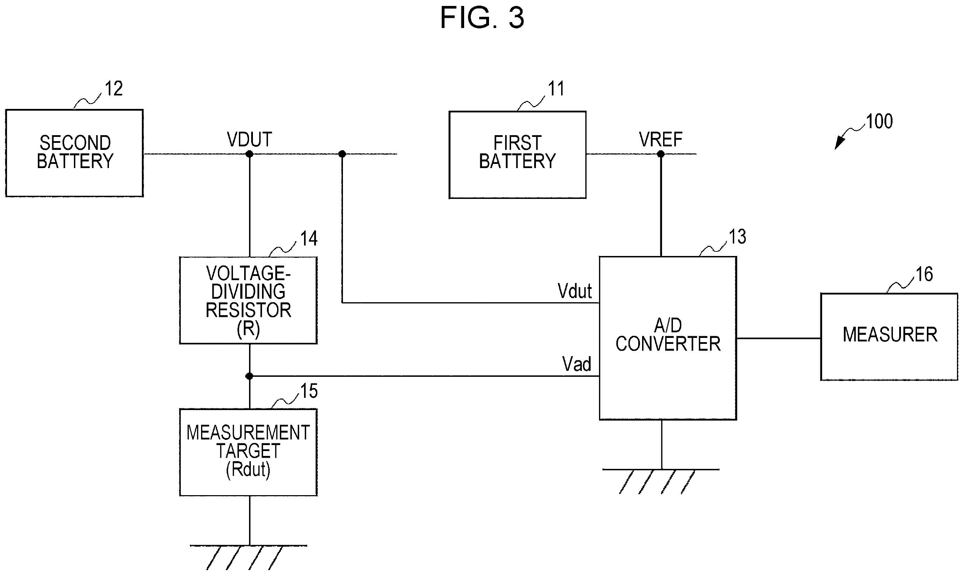

Accordingly, in the present embodiment, there is provided a mechanism that cancels out an influence of a variation in the reference voltage VREF and an influence of a variation in the voltage VDUT while supplying the reference voltage VREF and the voltage VDUT from different power supplies. Such a mechanism makes it possible to perform highly accurate A/D conversion while eliminating influences of variations in the reference voltage VREF and the voltage VDUT. In the present embodiment, the stability of operation regarding the power supply of the inhaler device 100 can be increased from the above viewpoint. Hereinafter, the present embodiment will be described with reference to Fig. 3 to Fig. 5.

(2) A/D conversion according to present embodiment

Fig. 3 is a diagram for describing a configuration related to A/D conversion of the inhaler device 100 according to the present embodiment. As illustrated in Fig. 3, the inhaler device 100 according to the present embodiment includes a first battery 11, a second battery 12, an analog-to-digital (A/D) converter 13, a voltage-dividing resistor 14, a measurement target 15, and a measurer 16. The first battery 11 and the second battery 12 are included in, for example, the power supply 111 illustrated in Fig. 1 and Fig. 2. The A/D converter 13, the voltage-dividing resistor 14, and the measurer 16 are included in, for example, the sensor 112 illustrated in Fig. 1 and Fig. 2.

The first battery 11 and the second battery 12 store electric power and supply the stored electric power to another structural element. The first battery 11 and the second battery 12 are separately configured. The reference voltage VREF that is to be applied to the A/D converter 13 is supplied from the first battery 11. The voltage VDUT that is to be applied to the circuit to be measured is supplied from the second battery 12. In the example illustrated in Fig. 3, the circuit to be measured includes the voltage-dividing resistor 14 and the measurement target 15.

The voltage-dividing resistor 14 is a resistor having a predetermined resistance R (i.e., a known resistance). The voltage-dividing resistor 14 is an example of a first resistor in the present embodiment. The voltage-dividing resistor 14 and the measurement target 15 form a so-called voltage-dividing circuit. The voltage-dividing circuit is a circuit that generates a voltage proportional to an input voltage at an intermediate point of a plurality of resistors.

The measurement target 15 is a target whose resistance Rdut is to be measured. The resistance Rdut of the measurement target 15 is variable. As illustrated in Fig. 3, the measurement target 15 is connected in series to the voltage-dividing resistor 14.

The A/D converter 13 outputs an A/D conversion value of a voltage applied thereto. The A/D converter 13 outputs an A/D conversion value of a voltage applied thereto in association with a reference voltage. The reference voltage is a voltage for determining the full scale of the A/D conversion value.

The reference voltage is applied to the A/D converter 13 from the first battery 11. In the example illustrated in Fig. 3, VREF is applied as the reference voltage. In response to the same voltage as the reference voltage VREF being applied, the A/D converter 13 outputs a value corresponding to the full scale (hereinafter also referred to as a full-scale value). In response to a voltage equal to or lower than the reference voltage VREF, an A/D conversion value equal to or smaller than the full-scale value is output. On the other hand, in response to a voltage exceeding the reference voltage VREF being applied to the A/D converter 13, the A/D conversion value is clipped to an upper limit value. That is, a full-scale value is output.

A power supply voltage Vdut is applied to the A/D converter 13 from the second battery 12. The A/D converter 13 outputs an A/D conversion value of the power supply voltage Vdut in the full scale based on the reference voltage VREF. Here, the voltage to be applied to the voltage-dividing resistor 14 from the second battery 12 is applied as the power supply voltage Vdut to the A/D converter 13. That is, the voltage before being divided by the voltage-dividing resistor 14 and the measurement target 15 is applied as the power supply voltage Vdut to the A/D converter 13. The power supply voltage Vdut is an example of a first voltage in the present embodiment. In the example illustrated in Fig. 3, the power supply voltage Vdut is equal to the voltage VDUT.

An input voltage Vad is applied to the A/D converter 13 from the second battery 12. The A/D converter 13 outputs an A/D conversion value of the input voltage Vad in the full scale based on the reference voltage VREF. Here, the voltage to be applied to the measurement target 15 from the second battery 12 via the voltage-dividing resistor 14 is applied as the input voltage Vad to the A/D converter 13. That is, a divided voltage at an intermediate point between the voltage-dividing resistor 14 and the measurement target 15 is applied as the input voltage Vad to the A/D converter 13. The input voltage Vad is an example of a second voltage in the present embodiment.

A change in the resistance Rdut of the measurement target 15 is reflected in the input voltage Vad. More specifically, because the resistance R of the voltage-dividing resistor 14 is known, when the input voltage Vad changes, a change in the resistance Rdut of the measurement target 15 can be grasped from a change in the A/D conversion value of the input voltage Vad.

The measurer 16 measures the resistance of the measurement target 15, based on an A/D conversion value output from the A/D converter 13. The measurer 16 is constituted by a micro control unit (MCU), for example. Specifically, the measurer 16 calculates the resistance Rdut of the measurement target 15, based on the ratio between the A/D conversion value of the input voltage Vad and the A/D conversion value of the power supply voltage Vdut, and on the resistance R of the voltage-dividing resistor 14. Hereinafter, a method for calculating the resistance Rdut of the measurement target 15 will be described in detail.

The measurer 16 is able to calculate the input voltage Vad from the A/D conversion value of the input voltage Vad. In a case where the A/D converter 13 has a resolution of 10 bits with a full-scale value of 1023 (= $2^{10}$ - 1), the input voltage Vad is calculated by the following equation.

[Math. 2]

$$Vad = \frac{\text{A/D conversion value } (Vad)}{2^{10}} \times VREF \qquad \cdots (2)$$

Here, the A/D conversion value (Vad) is the A/D conversion value of the input voltage Vad.

The measurer 16 is able to calculate the power supply voltage Vdut from the A/D conversion value of the power supply voltage Vdut. In a case where the A/D converter 13 has a resolution of 10 bits with a full-scale value of 1023 (= $2^{10}$ - 1), the power supply voltage Vdut is calculated by the following equation.

[Math. 3]

$$Vdut = \frac{\text{A/D conversion value } (Vdut)}{2^{10}} \times VREF \qquad \cdots (3)$$

Here, the A/D conversion value (Vdut) is the A/D conversion value of the power supply voltage Vdut.

Because the voltage-dividing resistor 14 and the measurement target 15 form a voltage-dividing circuit, the relationship expressed by the following equation is established between the input voltage Vad and the power supply voltage Vdut.

[Math. 4]

$$Vad = VDUT \times \frac{Rdut}{Rdut + R} \qquad \cdots (4)$$

The right side of the above equation (2) is substituted for Vad on the left side of the above equation (4), the right side of the above equation (3) is substituted for VDUT on the right side of above equation (4) because Vdut = VDUT, and thereby the above equation (4) is transformed through arrangement into the following equation.
[Math. 5]

$$Rdut = \frac{R \times \dfrac{\text{A/D conversion value } (Vad)}{\text{A/D conversion value } (Vdut)}}{1 - \dfrac{\text{A/D conversion value } (Vad)}{\text{A/D conversion value } (Vdut)}} \quad \cdots (5)$$

The measurer 16 calculates the resistance Rdut of the measurement target 15 by using the above equation (5). As expressed by the above equation (5), the resistance Rdut of the measurement target 15 is calculated, based on the ratio between the A/D conversion value of the input voltage Vad and the A/D conversion value of the power supply voltage Vdut. Thus, the measurer 16 is able to calculate the resistance Rdut of the measurement target 15 while eliminating influences of variations in the reference voltage VREF and the voltage VDUT. That is, the measurer 16 is able to calculate the resistance Rdut of the measurement target 15 with high accuracy.

In an example, it is assumed that the voltage VDUT is constant and the reference voltage VREF varies. In this case, the full scale of A/D conversion varies, and thus the A/D conversion value of the power supply voltage Vdut and the A/D conversion value of the input voltage Vad vary. However, even if the reference voltage VREF varies, the voltage division ratio of the voltage-dividing circuit formed of the voltage-dividing resistor 14 and the measurement target 15 does not vary, and thus the ratio between the A/D conversion value of the power supply voltage Vdut and the A/D conversion value of the input voltage Vad does not vary. Thus, the resistance Rdut of the measurement target 15 calculated by the above equation (5) does not vary. That is, the measurer 16 is able to calculate the resistance Rdut of the measurement target 15 while eliminating an influence of a variation in the reference voltage VREF.

In another example, it is assumed that the reference voltage VREF is constant and the voltage VDUT varies. In this case, the power supply voltage Vdut and the input voltage Vad vary, and thus the A/D conversion value of the power supply voltage Vdut and the A/D conversion value of the input voltage Vad vary. However, even if the voltage VDUT varies, the voltage division ratio of the voltage-dividing circuit formed of the voltage-dividing resistor 14 and the measurement target 15 does not vary, and thus the ratio between the A/D conversion value of the power supply voltage Vdut and the A/D conversion value of the input voltage Vad does not vary. Thus, the resistance Rdut of the measurement target 15 calculated by the above equation (5) does not vary. That is, the measurer 16 is able to calculate the resistance Rdut of the measurement target 15 while eliminating an influence of a variation in the voltage VDUT.

The absolute value of the power supply voltage Vdut is equal to or smaller than the absolute value of the reference voltage VREF. If the absolute value of the power supply voltage Vdut exceeds the absolute value of the reference voltage VREF, the power supply voltage Vdut overflows with respect to the full scale of A/D conversion, and the A/D conversion value is clipped to the upper limit value. That is, the A/D conversion value of the power supply voltage Vdut becomes inaccurate. In this regard, if the absolute value of the power supply voltage Vdut is equal to or smaller than the absolute value of the reference voltage VREF, the power supply voltage Vdut falls within the full scale of A/D conversion. Thus, clipping of the A/D conversion value to the upper limit value is avoided. Thus, an accurate A/D conversion value of the power supply voltage Vdut can be obtained.

From the above-described point of view, the inhaler device 100 may further include a corrector that performs correction such that the absolute value of the power supply voltage Vdut is equal to or smaller than the absolute value of the reference voltage VREF. In an example, the corrector is disposed between the second battery 12 and the A/D converter 13, and divides or attenuates the voltage VDUT so that the absolute value of the power supply voltage Vdut is equal to or smaller than the absolute value of the reference voltage VREF. With this configuration, the absolute value of the power supply voltage Vdut can be made equal to or smaller than the absolute value of the reference voltage VREF, and thus an accurate A/D conversion value of the power supply voltage Vdut can be obtained. As a result, the measurer 16 is able to calculate the resistance Rdut of the measurement target 15 with high accuracy.

The corrector may be a resistor having a predetermined resistance. The resistor is an example of a second resistor in the present embodiment. For example, the first resistor (i.e., the voltage-dividing resistor 14) and the second resistor may constitute a voltage-dividing circuit, and a divided voltage at an intermediate point between the first resistor and the second resistor may be applied as the power supply voltage Vdut to the A/D converter 13. In this case, however, it is desired that calibration be performed in advance so that no error occurs between a calculated voltage division ratio calculated from the resistance of the first resistor and the resistance of the second resistor and an actual voltage division ratio. This is because, if there is an error between the calculated voltage division ratio and the actual voltage division ratio, the power supply voltage Vdut calculated from the A/D conversion value of the power supply voltage Vdut has a deviated value. To perform the calibration herein, the circuit may be changed so that the calculated voltage division ratio and the actual voltage division ratio coincide with each other, or the actual voltage division ratio may be used at the time of calculating a voltage from the A/D conversion value. The number of resistors serving as a corrector, and the configuration of the voltage-dividing circuit such as the position of the intermediate point are not particularly limited as long as a desired voltage division ratio can be realized.

Alternatively, the corrector may be an amplifier that attenuates an input voltage and outputs the attenuated voltage. In any case, providing of the corrector enables an accurate A/D conversion value of the power supply voltage Vdut to be obtained.

(3) Temperature measurement

The measurement target 15 is a member whose electric resistance changes in accordance with a temperature change. Thus, the measurer 16 measures the temperature of the measurement target 15, based on the resistance of the measurement target 15. This configuration makes it possible to measure the temperature of the measurement target 15.

The measurement target 15 is the heater 121. With this configuration, the measurer 16 is able to measure the temperature of the heater 121, based on the resistance of the heater 121. In addition, it is not necessary to separately provide a thermistor or the like to measure the temperature of the heater 121, and thus design easiness of the inhaler device 100 is improved.

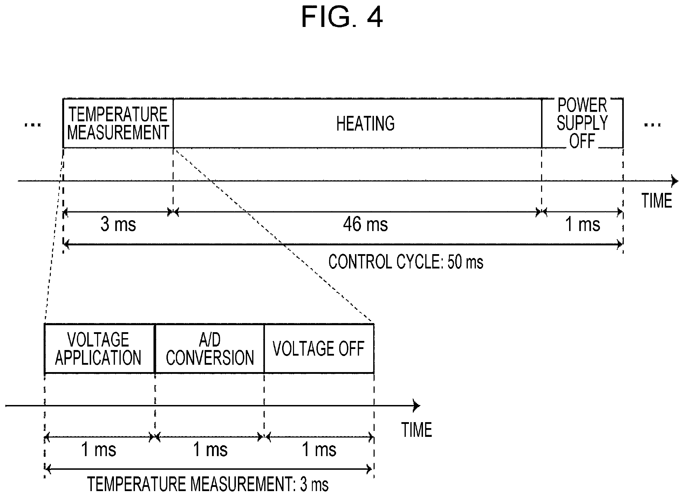

Supply of electric power to the measurement target 15, for measuring the resistance of the measurement target 15, is periodically executed at a first timing. That is, supply of electric power to the heater 121, for measuring the temperature of the heater 121, is periodically executed at a first timing. On the other hand, supply of electric power to the heater 121, for heating the aerosol source by the heater 121, is periodically executed at a second timing different from the first timing. With this configuration, temperature measurement and heating are alternately executed. Thus, it is possible to continue heating by the heater 121 while monitoring the temperature of the heater 121. At the time of temperature measurement, a voltage weaker than that applied at the time of heating may be applied to the heater 121. This configuration makes it possible to reduce power consumption. The configuration also makes it possible to prevent a failure of the measurement system resulting from flowing of a large current. The timing to supply electric power to the heater 121 will be described in detail with reference to Fig. 4.

Fig. 4 is a diagram for describing an example of a timing to supply electric power to the heater 121 in the inhaler device 100 according to the present embodiment. The horizontal axis in Fig. 4 is a time axis, and time flows from left to right. The inhaler device 100 repeatedly executes the process illustrated in Fig. 4, with a control cycle of 50 ms being one cycle. As illustrated in Fig. 4, in the first 3 ms in the control cycle, supply of electric power to the heater 121, for measuring the temperature of the heater 121, is executed. In the subsequent 46 ms, supply of electric power to the heater 121 for heating is executed. In the subsequent 1 ms, the supply of electric power to the heater 121 is stopped.

The details of the first 3 ms in the control cycle will be described in detail. As illustrated in Fig. 4, in the first 1 ms, each of the first battery 11 and the second battery 12 applies a voltage. At this time, as described with reference to Fig. 3, the first battery 11 applies the reference voltage VREF to the A/D converter 13, and the second battery 12 applies the power supply voltage Vdut and the input voltage Vad to the A/D converter 13. Subsequently, in the intermediate 1 ms, voltage application by each of the first battery 11 and the second battery 12 continues, and A/D conversion is performed. The intermediate 1 ms includes a settling time of the A/D converter 13. In the last 1 ms, the supply of electric power by the first battery 11 and the second battery 12 is stopped. In the last 1 ms, the A/D converter 13 is discharged.

(4) Flow of process

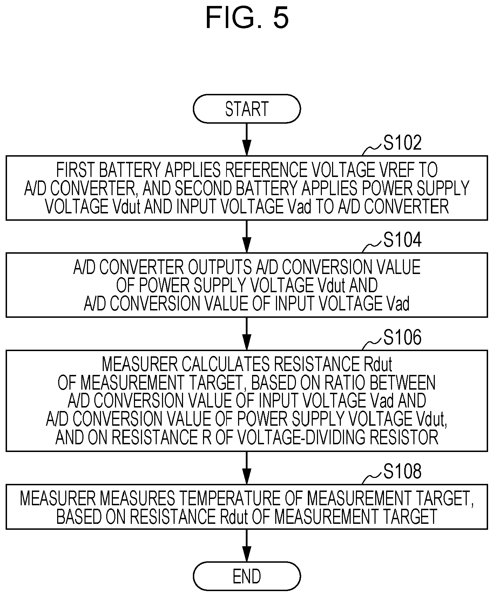

Fig. 5 is a flowchart illustrating an example of a flow of a process executed by the inhaler device 100 according to the present embodiment.

As illustrated in Fig. 5, first, the first battery 11 applies the reference voltage VREF to the A/D converter 13, and the second battery 12 applies the power supply voltage Vdut and the input voltage Vad to the A/D converter 13 (step S102).

Subsequently, the A/D converter 13 outputs an A/D conversion value of the power supply voltage Vdut and an A/D conversion value of the input voltage Vad (step S104).

Subsequently, the measurer 16 calculates the resistance Rdut of the measurement target 15, based on the ratio between the A/D conversion value of the input voltage Vad and the A/D conversion value of the power supply voltage Vdut, and on the resistance R of the voltage-dividing resistor 14 (step S106).

Subsequently, the measurer 16 measures the temperature of the measurement target 15, based on the resistance Rdut of the measurement target 15 (step S108).

(5) Effects of inhaler device according to present embodiment

Hereinafter, effects of the inhaler device 100 according to the present embodiment will be described with reference to a comparative example.

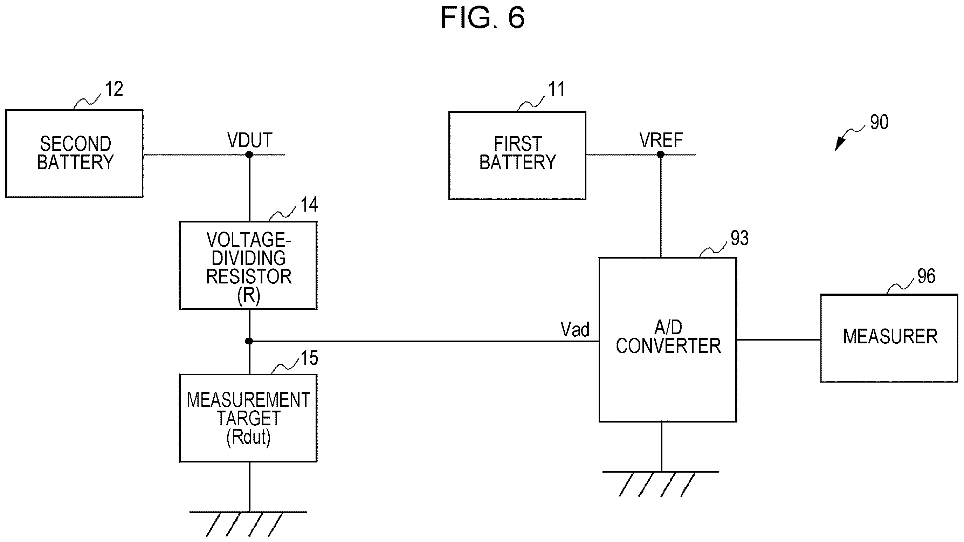

Fig. 6 is a diagram for describing a configuration related to A/D conversion of an inhaler device 90 according to the comparative example. As illustrated in Fig. 6, the inhaler device 90 according to the comparative example includes a first battery 11, a second battery 12, an A/D converter 93, a voltage-dividing resistor 14, a measurement target 15, and a measurer 96.

The configurations of the first battery 11, the second battery 12, the voltage-dividing resistor 14, and the measurement target 15 are the same as the configurations of the corresponding structural elements in the inhaler device 100 according to the present embodiment illustrated in Fig. 3. When Fig. 3 and Fig. 6 are compared with each other, in the inhaler device 90 according to the comparative example, the power supply voltage Vdut is not applied to the A/D converter 93.

Thus, the A/D converter 93 outputs an A/D conversion value of the input voltage Vad in the full scale based on the reference voltage VREF. The measurer 96 measures the resistance Rdut of the measurement target 95, based on the A/D conversion value of the input voltage Vad.

In the inhaler device 90 according to the comparative example, the above equation (2) and the above equation (4) are obtained. The right side of the above equation (2) is substituted for Vad on the left side of the above equation (4), and thereby the above equation (4) is transformed through arrangement into the following equation.
[Math. 6]

$$Rdut = \frac{R \times \dfrac{\text{A/D conversion value }(Vad)}{2^{10}} \times \dfrac{VREF}{VDUT}}{1 - \dfrac{\text{A/D conversion value }(Vad)}{2^{10}} \times \dfrac{VREF}{VDUT}} \quad \cdots (\,6\,)$$

The measurer 96 calculates the resistance Rdut of the measurement target 15 by using the above equation (6). As expressed by the above equation (6), in the comparative example, influences of variations in the reference voltage VREF and the voltage VDUT appear in the calculation result of the resistance Rdut of the measurement target 15. Thus, in the inhaler device 90 according to the comparative example, it is difficult to eliminate influences of variations in the reference voltage VREF and the power supply voltage Vdut.

In an example, it is assumed that the voltage VDUT is constant and the reference voltage VREF varies. In this case, the full scale of A/D conversion varies, and thus the A/D conversion value of the input voltage Vad varies. However, it is difficult for the measurer 96 to identify whether the variation in the A/D conversion value of the input voltage Vad is caused by an influence of a variation in the voltage VDUT or an influence of a variation in the reference voltage VREF. Thus, it is difficult for the measurer 96 to eliminate the influence of the variation in the reference voltage VREF.

In another example, it is assumed that the reference voltage VREF is constant and the voltage VDUT varies. In this case, the input voltage Vad varies, and thus the A/D conversion value of the input voltage Vad varies. However, it is difficult for the measurer 96 to identify whether the variation in the A/D conversion value of the input voltage Vad is caused by an influence of a variation in the voltage VDUT or an influence of a variation in the reference voltage VREF. Thus, it is difficult for the measurer 96 to eliminate the influence of the variation in the voltage VDUT.

As described above, in the inhaler device 90 according to the comparative example, it is difficult to eliminate influences of variations in the reference voltage VREF and the power supply voltage Vdut. Thus, the inhaler device 90 according to the comparative example may erroneously grasp variations in the reference voltage VREF and the power supply voltage Vdut as a variation in the resistance Rdut of the measurement target 15. Alternatively, the inhaler device 90 according to the comparative example may ignore a variation in the resistance Rdut of the measurement target 15, which should be originally grasped, as influences of variations in the reference voltage VREF and the power supply voltage Vdut.

On the other hand, in the inhaler device 100 according to the present embodiment, as described above with reference to Fig. 3, it is possible to calculate the resistance Rdut of the measurement target 15 while eliminating influences of variations in the reference voltage VREF and the voltage VDUT. That is, the inhaler device 100 according to the present embodiment is able to calculate the resistance Rdut of the measurement target 15 with high accuracy, compared with the inhaler device 90 according to the comparative example.

<<3. Supplementary description>>

While a preferred embodiment of the present invention has been described in detail with reference to the accompanying drawings, the present invention is not limited to the foregoing example. It will be apparent that those skilled in the art to which the present invention belongs are able to conceive of various modifications or variations within the scope of the technical ideas described in the claims, and it is understood that such modifications or variations also belong to the technical scope of the present invention.

For example, in the above-described embodiment, an example is described in which the measurement target 15 is the heater 121, but the present invention is not limited to this example. The measurement target 15 may be a thermistor. The thermistor is a member whose electric resistance largely changes in accordance with a temperature change. The thermistor is disposed near the heater 121, for example, adjacent to the heater 121. In this case, the temperature of the thermistor changes in accordance with a temperature change of the heater 121. With this configuration, the measurer 16 is able to measure the temperature of the thermistor, based on the resistance of the thermistor, and measure the temperature of the heater 121, based on the measured temperature of the thermistor.

A series of processes performed by the individual devices described in this specification may be implemented by using any of software, hardware, and a combination of software and hardware. Programs constituting the software are stored in advance in, for example, a recording medium (non-transitory medium) provided inside or outside each device. Each program is read into a RAM and is executed by a processor such as a CPU when being executed by a computer that controls each device described in this specification, for example. The recording medium is, for example, a magnetic disk, an optical disc, a magnetooptical disc, a flash memory, or the like. In addition, the foregoing computer program may be distributed via a network, for example, without using a recording medium.

In addition, the process described using a flowchart in this specification need not necessarily be executed in the illustrated order. Some processing steps may be executed in parallel. In addition, an additional processing step may be employed, and some processing steps may be omitted.

The following configurations also belong to the technical scope of the present invention.

(1) An inhaler device configured to generate an aerosol, the inhaler device including:

a first resistor having a predetermined resistance;
a measurement target connected in series to the first resistor and having a resistance that is variable;
an analog-to-digital (A/D) converter configured to output an A/D conversion value of a voltage applied thereto; and
a measurer configured to measure the resistance of the measurement target, based on the A/D conversion value output from the A/D converter, wherein
the A/D converter is configured to

receive a reference voltage from a first power supply, the reference voltage being a voltage determining a full scale of an A/D conversion value,
output an A/D conversion value of a first voltage applied from a second power supply different from the first power supply, and
output an A/D conversion value of a second voltage applied to the measurement target from the second power supply via the first resistor, and

the measurer is configured to calculate the resistance of the measurement target, based on a ratio between the A/D conversion value of the first voltage and the A/D conversion value of the second voltage, and on the resistance of the first resistor.

(2) The inhaler device according to (1) above, wherein

the measurer is configured to calculate the resistance of the measurement target by using an equation below:
[Math. 7]

$$Rdut = \frac{R \times \frac{\text{A/D conversion value } (Vad)}{\text{A/D conversion value } (Vdut)}}{1 - \frac{\text{A/D conversion value } (Vad)}{\text{A/D conversion value } (Vdut)}} \quad \cdots (7)$$

where A/D conversion value (Vdut) is the A/D conversion value of the first voltage, A/D conversion value (Vad) is the A/D conversion value of the second voltage, R is the resistance of the first resistor, and Rdut is the resistance of the measurement target.

(3) The inhaler device according to (1) or (2) above, wherein
the first voltage has an absolute value that is equal to or smaller than an absolute value of the reference voltage.
(4) The inhaler device according to (3) above, further including:
a corrector configured to perform correction such that the absolute value of the first voltage is equal to or smaller than the absolute value of the reference voltage.

(5) The inhaler device according to (4) above, wherein
the corrector is a second resistor having a predetermined resistance.

(6) The inhaler device according to (4) above, wherein
the corrector is an amplifier configured to attenuate a voltage input thereto and output the attenuated voltage.

(7) The inhaler device according to any one of (1) to (6) above, wherein
the A/D converter is configured to output the A/D conversion value of the first voltage applied to the first resistor from the second power supply.

(8) The inhaler device according to any one of (1) to (7) above, wherein
the measurer is configured to measure a temperature of the measurement target, based on the resistance of the measurement target.

(9) The inhaler device according to any one of (1) to (8) above, further including:

> a heater configured to heat an aerosol source to generate the aerosol, wherein
> the measurement target is the heater.

(10) The inhaler device according to any one of (1) to (9) above, further including:

> a heater configured to heat an aerosol source to generate the aerosol; and
> a thermistor having a temperature that changes in accordance with a temperature change of the heater, wherein
> the measurement target is the thermistor.

(11) The inhaler device according to (9) or (10) above, wherein

> supply of electric power to the measurement target, for measuring the resistance of the measurement target, is periodically executed at a first timing, and
> supply of electric power to the heater, for heating the aerosol source by the heater, is periodically executed at a second timing different from the first timing.

(12) A control method for controlling an inhaler device configured to generate an aerosol,

> the inhaler device including:

> > a first resistor having a predetermined resistance;
> > a measurement target connected in series to the first resistor and having a resistance that is variable; and
> > an analog-to-digital (A/D) converter configured to output an A/D conversion value of a voltage applied thereto,

> the control method including:

> > applying a reference voltage to the A/D converter from a first power supply, the reference voltage being a voltage determining a full scale of an A/D conversion value;
> > applying a first voltage to the A/D converter from a second power supply different from the first power supply and causing the A/D converter to output an A/D conversion value of the first voltage;
> > applying a second voltage to the measurement target from the second power supply via the first resistor and causing the A/D converter to output an A/D conversion value of the second voltage; and
> > calculating the resistance of the measurement target, based on a ratio between the A/D conversion value of the first voltage and the A/D conversion value of the second voltage, and on the resistance of the first resistor.

Reference Signs List

100    inhaler device
110    power supply unit
111    power supply
112    sensor
113    notifier

114  memory
115  communicator
116  controller
120  cartridge
121  heater
122  liquid guide
123  liquid storage
124  mouthpiece
130  flavor imparting cartridge
131  flavor source
140  holder
141  internal space
142  opening
143  bottom
144  heat insulator
150  stick substrate
151  substrate
152  inhalation port
180  airflow path
181  air inlet hole
182  air outlet hole
11   first battery
12   second battery
13   A/D converter
14   voltage-dividing resistor
15   measurement target
16   measurer

## Claims

1. An inhaler device configured to generate an aerosol, the inhaler device comprising:

   a first resistor having a predetermined resistance;
   a measurement target connected in series to the first resistor and having a resistance that is variable;
   an analog-to-digital (A/D) converter configured to output an A/D conversion value of a voltage applied thereto; and
   a measurer configured to measure the resistance of the measurement target, based on the A/D conversion value output from the A/D converter, wherein
   the A/D converter is configured to

   receive a reference voltage from a first power supply, the reference voltage being a voltage determining a full scale of an A/D conversion value,
   output an A/D conversion value of a first voltage applied from a second power supply different from the first power supply, and
   output an A/D conversion value of a second voltage applied to the measurement target from the second power supply via the first resistor, and

   the measurer is configured to calculate the resistance of the measurement target, based on a ratio between the A/D conversion value of the first voltage and the A/D conversion value of the second voltage, and on the resistance of the first resistor.

2. The inhaler device according to claim 1, wherein

   the measurer is configured to calculate the resistance of the measurement target by using an equation below:
   [Math. 1]

$$Rdut = \frac{R \times \dfrac{\text{A/D conversion value } (Vad)}{\text{A/D conversion value } (Vdut)}}{1 - \dfrac{\text{A/D conversion value } (Vad)}{\text{A/D conversion value } (Vdut)}} \qquad \cdots (1)$$

,

where A/D conversion value (Vdut) is the A/D conversion value of the first voltage, A/D conversion value (Vad) is the A/D conversion value of the second voltage, R is the resistance of the first resistor, and Rdut is the resistance of the measurement target.

3. The inhaler device according to claim 1 or 2, wherein
the first voltage has an absolute value that is equal to or smaller than an absolute value of the reference voltage.

4. The inhaler device according to claim 3, further comprising:
a corrector configured to perform correction such that the absolute value of the first voltage is equal to or smaller than the absolute value of the reference voltage.

5. The inhaler device according to claim 4, wherein
the corrector is a second resistor having a predetermined resistance.

6. The inhaler device according to claim 4, wherein
the corrector is an amplifier configured to attenuate a voltage input thereto and output the attenuated voltage.

7. The inhaler device according to any one of claims 1 to 6, wherein
the A/D converter is configured to output the A/D conversion value of the first voltage applied to the first resistor from the second power supply.

8. The inhaler device according to any one of claims 1 to 7, wherein
the measurer is configured to measure a temperature of the measurement target, based on the resistance of the measurement target.

9. The inhaler device according to any one of claims 1 to 8, further comprising:

a heater configured to heat an aerosol source to generate the aerosol, wherein
the measurement target is the heater.

10. The inhaler device according to any one of claims 1 to 9, further comprising:

a heater configured to heat an aerosol source to generate the aerosol; and
a thermistor having a temperature that changes in accordance with a temperature change of the heater, wherein
the measurement target is the thermistor.

11. The inhaler device according to claim 9 or 10, wherein

supply of electric power to the measurement target, for measuring the resistance of the measurement target, is periodically executed at a first timing, and
supply of electric power to the heater, for heating the aerosol source by the heater, is periodically executed at a second timing different from the first timing.

12. A control method for controlling an inhaler device configured to generate an aerosol,

the inhaler device comprising:

a first resistor having a predetermined resistance;
a measurement target connected in series to the first resistor and having a resistance that is variable; and
an analog-to-digital (A/D) converter configured to output an A/D conversion value of a voltage applied thereto,

the control method comprising:

applying a reference voltage to the A/D converter from a first power supply, the reference voltage being a voltage determining a full scale of an A/D conversion value;

applying a first voltage to the A/D converter from a second power supply different from the first power supply and causing the A/D converter to output an A/D conversion value of the first voltage;

applying a second voltage to the measurement target from the second power supply via the first resistor and causing the A/D converter to output an A/D conversion value of the second voltage; and

calculating the resistance of the measurement target, based on a ratio between the A/D conversion value of the first voltage and the A/D conversion value of the second voltage, and on the resistance of the first resistor.

FIG. 1

100A

110 POWER SUPPLY UNIT

116A CONTROLLER

111A POWER SUPPLY
112A SENSOR
113A NOTIFIER
114A MEMORY
115A COMMUNICATOR

AIR

181

120 CARTRIDGE
122 121A
123
121A

LIQUID STORAGE

130 FLAVOR IMPARTING CARTRIDGE
131 FLAVOR SOURCE

124

180

182

190

181

AIR

# FIG. 2

EP 4 212 037 A1

# FIG. 3

# FIG. 4

··· | TEMPERATURE MEASUREMENT | HEATING | POWER SUPPLY OFF | ···

3 ms     46 ms     1 ms    TIME

CONTROL CYCLE: 50 ms

VOLTAGE APPLICATION | A/D CONVERSION | VOLTAGE OFF

1 ms    1 ms    1 ms    TIME

TEMPERATURE MEASUREMENT: 3 ms

# FIG. 5

START

↓

S102

FIRST BATTERY APPLIES REFERENCE VOLTAGE VREF TO
A/D CONVERTER, AND SECOND BATTERY APPLIES POWER SUPPLY
VOLTAGE Vdut AND INPUT VOLTAGE Vad TO A/D CONVERTER

↓

S104

A/D CONVERTER OUTPUTS A/D CONVERSION VALUE
OF POWER SUPPLY VOLTAGE Vdut AND
A/D CONVERSION VALUE OF INPUT VOLTAGE Vad

↓

S106

MEASURER CALCULATES RESISTANCE Rdut
OF MEASUREMENT TARGET, BASED ON RATIO BETWEEN
A/D CONVERSION VALUE OF INPUT VOLTAGE Vad AND
A/D CONVERSION VALUE OF POWER SUPPLY VOLTAGE Vdut,
AND ON RESISTANCE R OF VOLTAGE-DIVIDING RESISTOR

↓

S108

MEASURER MEASURES TEMPERATURE OF MEASUREMENT TARGET,
BASED ON RESISTANCE Rdut OF MEASUREMENT TARGET

↓

END

## FIG. 6

EP 4 212 037 A1

## INTERNATIONAL SEARCH REPORT

| International application No. |
|---|
| PCT/JP2020/046702 |

| A. CLASSIFICATION OF SUBJECT MATTER |
|---|
| A24F 40/50(2020.01)i |
| FI: A24F40/50 |
| |
| According to International Patent Classification (IPC) or to both national classification and IPC |

| B. FIELDS SEARCHED |
|---|
| Minimum documentation searched (classification system followed by classification symbols) |
| A24F40/50 |

| Documentation searched other than minimum documentation to the extent that such documents are included in the fields searched |  |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

| Electronic data base consulted during the international search (name of data base and, where practicable, search terms used) |
|---|
|  |

## C. DOCUMENTS CONSIDERED TO BE RELEVANT

| Category* | Citation of document, with indication, where appropriate, of the relevant passages | Relevant to claim No. |
|---|---|---|
| X | WO 2019/146063 A1 (JAPAN TOBACCO INC.) 01 August | 1–9, 11–12 |
| A | 2019 (2019-08-01) paragraphs [0167]–[0180], fig. 1–7 | 10 |
| A | JP 6761913 B1 (JAPAN TOBACCO INC.) 30 September 2020 (2020-09-30) entire text, all drawings | 1–12 |
| A | JP 2020-115738 A (JAPAN TOBACCO INC.) 30 July 2020 (2020-07-30) entire text, all drawings | 1–12 |

| ☐ Further documents are listed in the continuation of Box C. | ☒ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 February 2021 (12.02.2021) | 22 February 2021 (22.02.2021) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

| Patent Documents referred in the Report | Publication Date | Patent Family | Publication Date |
|---|---|---|---|
| WO 2019/146063 A1 | 01 Aug. 2019 | (Family: none) | |
| JP 6761913 B1 | 30 Sep. 2020 | (Family: none) | |
| JP 2020-115738 A | 30 Jul. 2020 | (Family: none) | |

**INTERNATIONAL SEARCH REPORT**
Information on patent family members

International application No.
PCT/JP2020/046702

Form PCT/ISA/210 (patent family annex) (January 2015)

**REFERENCES CITED IN THE DESCRIPTION**

**Patent documents cited in the description**

- JP 2020504599 A